# EUROPEAN PATENT APPLICATION

(11) **EP 0 811 355 A1**
(43) Date of publication of application: **10.12.1997**
(21) Application number: 96304009.2
(22) Date of filing: 03.06.1996
(51) Int. Cl.: A61B 17/30, B25B 9/02

(54) **Tool with magnifying lens**

(71) Applicant: Richardson, Ramon, Frederick, Chessington, Surrey KT9 2JA (GB)
(72) Inventor: Richardson, Ramon, Frederick, Chessington, Surrey KT9 2JA (GB)
(74) Representative: Jehan, Robert

(57) **Abstract**

A magnifying tool (10) comprises a tubular support member (14) which includes first and second elongate cavities (18) at opposing sides of a first end (16) of the support member (14), first and second tool arms (20) insertable into and securable in the first and second cavities (18) respectively, and a magnifying lens (22) supported by the tubular support member (14) and having its point of focus oriented along the axis of the tubular support member (14). The tool arms (20) may be removed from the tool (10). Another embodiment provides a unitary tool (30) with pointed arm tips (36) for medical purposes.

## Description

The present invention relates to a magnifying tool and to a medical gripping tool.

US-A-3,774,883 discloses a tweezer assembly which includes a pair of tweezer legs supported at opposite sides of an annular support. The annular support includes a shoulder against which a magnifying lens can rest and be fixed. In an embodiment, the assembly includes a separate lens holder which can slide within an extended annular support so as to provide an adjustable focus point of the lens.

In medical fields, it is known to have disposable tweezers which are used once and then discarded. As a result of manufacturing costs and sterilisation problems the tweezers are generally as simple as possible, with a separate reusable handheld lens being provided for magnification purposes.

The present invention seeks to provide an improved magnifying tool and an improved medical gripping tool.

According to an aspect of the present invention, there is provided a magnifying tool as specified in claim 1.

Preferably, the first and second cavities and/or the first and second arms of the tool include releasable fastening means for allowing the first and second arms to be removed from the tubular support. The fastening means may include a burr and a slot on respective ones of the tool arms and elongate cavities, or any other suitable releasable fastening means.

Alternatively, the first and second tool arms may be permanently fixable in their respective cavities.

The lens is preferably securable to a second end of the tubular support, opposite to the first end. The lens and tool body may be moulded as a single unit.

In a preferred embodiment, the tubular support is formed from inner and outer tubular members, the inner member being insertable into the outer member; one of the inner and outer members including two elongate channels therein which, when the inner and outer members are assembled, form the first and second elongate cavities with a surface of the other of the inner and outer tubular members. The channels are preferably formed in the inner tubular member.

Preferably, the inner tubular member is shorter than the outer tubular member and, when inserted in the outer tubular member provides a shoulder delimited by a portion of an inner wall of the second end of the outer tubular member. The shoulder and inner wall of the outer tubular member serve to provide securing means for the lens.

The tool arms may, for example, be tweezer arms, pincer arms or arms of any other gripping tool. The ends of the arms may be pointed so as to enable them to pierce a surface worked upon by the tool, such as skin or the like.

According to another aspect of the present invention, there is provided a medical gripping tool as specified in claim 11.

The medical gripping tool can have a number of advantages, for example: (a) can be disposed of after a single use, thereby avoiding the inconvenience of re-sterilisation, (b) can pierce skin or the like before gripping, for example for removing lice, ticks or splinters; and (c) can magnify the region around the ends of the gripper arms, whereas in prior art medical tweezers it was necessary to provide separate magnifying means.

The body member and gripper arms are preferably moulded from a single moulding. The same preferably applies to the lens, which may be moulded integrally with the remainder of the tool.

Advantageously, the gripper arms are in the form of pivotless extensions of the body member.

The ends of the gripper arms may be in the form of tweezers and may have substantially flat opposing faces for assisting in gripping.

The body member may have a recessed annular portion at its second end for receiving a lens.

The gripper tool is preferably made of a plastics material.

In practice, a plurality of different versions of gripper tool would be provided, having different length arms. The arms may range from approximately 5 to approximately 10 centimetres, the lens of each tool being selected so as to have a point of focus at the ends of the gripper arms. Thus, depending upon the application, a tool with suitable length arms can be chosen, without any need to adjust lenses or the like.

It is envisaged that the tool would be provided in a sealed package already sterilised, for example by irradiation, such that a package containing the desired tool can be selected, the package opened and tool immediately used. Following usage, the tool is immediately discarded.

An embodiment of the present invention is described below, by way of example only, with reference to the accompanying drawing, in which:
Figure 1 is a cross-sectional view of an embodiment of magnifying tool;
Figure 2 is a cross-sectional view of the tool of Figure 1, taken along line 2-2 of Figure 1; and
Figure 3 is a schematic view of an embodiment of medical gripping tool located within an embodiment of moulding device.

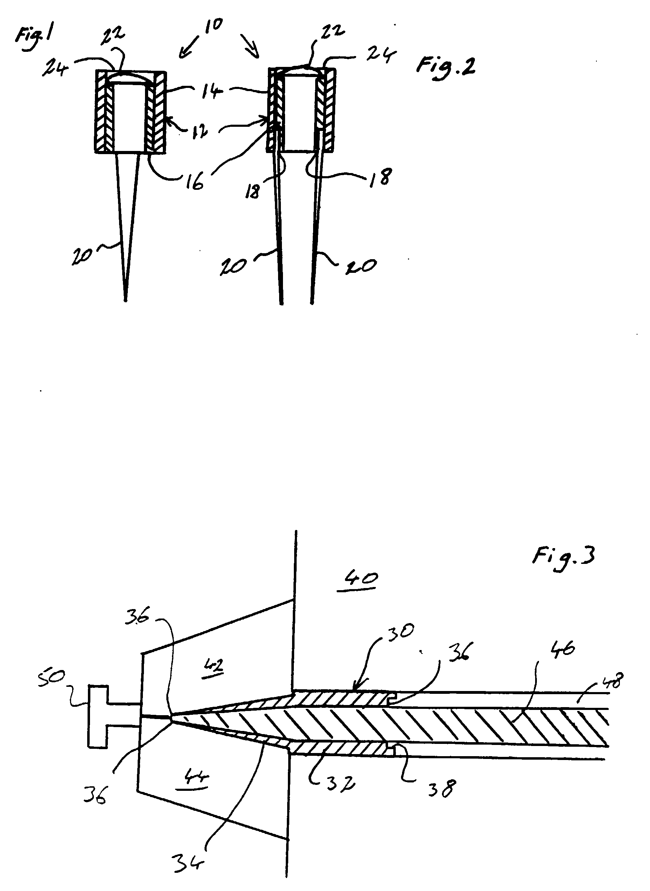

Referring to Figures 1 and 2, the embodiment of magnifying tweezers 10 shown includes a tubular support member 12 which is, in this example, substantially cylindrical and circular in plan view. The support member 12 is formed from inner and outer tubular members 14,16 which are a tight sliding fit one within the other, such that when fitted together they are substantially unitary. The inner and outer members may be secured together by other means, such as adhesive or the like.

The outer member 14 is substantially even and has a substantially smooth inner surface. On the other hand, the inner member 16 has formed in its outer surface a pair of channels 18 of rectangular cross-section. The channels 18 are disposed substantially at opposite ends of a diameter of the inner tubular member 16. As can be seen in Figure 2, when the inner tubular member 16 is fitted into the outer tubular member 14, the top of the channels 18 are closed by the inner surface of the outer tubular member 14, thereby to provide two elongate cavities having their openings at a first end of the tubular support member 12 and extending to a predetermined point provided by the ends of the channels 18.

Fitting within each elongate cavity is a coupling end of a tweezer arm 20. The free end of each tweezer arm is, in this example, pointed, although may be of any other suitable form. The tweezer arms may be fixed within the elongate cavities, for example by means of adhesive or suitable cementing substance, by a suitable latch formed in the cavities or the coupling ends of the tweezer arms 20, or by any other suitable means.

Alternatively, the tweezer arms may be removable for the tubular support member 12 so that they may, for example, be replaced by other tool arms, of any desired type. In this embodiment, there may be provided on the coupling ends of the tool arms and in the elongate cavities (for example in the channels or on the inner surface of the outer tubular member 14) a slot and engaging burr or any other suitable releasable fastening means. On the other hand, should a suitably tight fit of the coupling ends of the tool arms in the cavities be provided, no other fastening means may be necessary.

As can be seen in Figures 1 and 2, at the end of the tubular support member 12 opposite the tweezer arms 20, there is provided a magnifying lens 22 formed from glass, perspex or other suitable transparent material. The lens 22 is oriented so as to have its point of focus directed substantially along the axis of the tubular member 12 and has a coefficient of refraction such that it is focused onto the region around the tips of the tweezer arms 20.

The inner tubular member 16 is slightly shorter than the outer tubular member 14, such that when they are fitted together, the inner tubular member 16, at the second end 24 of the support 12, is slightly recessed into the outer tubular member 14. In this manner, the inner tubular member 16 provides an annular shoulder delimited by the inner surface of the outer tubular member 14. The lens 22 rests on the annular shoulder and is preferably of such a size as to be a tight fit against the inner surface of the outer tubular member 14.

If necessary, the lens 22 is fixed into the tubular support member 12, for example by adhesive or by deformation of the wall of the outer tubular member 14 to provide restraining notches or the like. Alternatively, the lens 22 could be removable so as to be replaced with a lens of different focal point, for example should the tool 10 have tool arms of different lengths.

In another embodiment, the lens 22 is moulded integrally with the body of the tool, for example with either or both of the inner and outer tubular members.

It will be apparent that the tubular support member 12 could be made as a single piece of material, rather by two members as shown in Figures 1 and 2.

Referring to Figure 3, there is shown another embodiment of tweezer, in this case a plastics medical gripping tool 30 during its manufacture.

The medical gripping tool 30 includes a tubular body member 32 and a pair of gripper arms 34 which extend from opposing sides of a first end of the body member 32. The arms 34 are unitary with the body member 32 and have pointed ends 36. In the embodiment shown, the gripper arms 34 are in the form of pivotless extensions of the body member 32.

The pointed ends 36 of the gripper arms 34 are intended to be able to pierce skin or the like before gripping, for example for removing lice, ticks or splinters, while the lens magnifies the region around the ends 36 of the gripper arms 34. In order to assist in gripping, the ends of the gripper arms are in the form of tweezers, having substantially flat opposing faces for assisting in gripping.

The body member 32 is substantially cylindrical and circular in plan view. At its end opposite the gripper arms 34, the body member 32 is provided with a recessed annular shoulder 36 extending transverse to the axis of the body member 32 and delimited by an annular wall 38 at right angles thereto. It will be apparent that the annular shoulder 36 and wall 38 have the function of holding a lens (not shown) therein in such a manner that the focal point of the lens is directed along the axis of the tool 30.

The lens may be moulded integrally with the tool body 30.

The medical gripping tool 30 is designed so as to be disposable after a single use, thereby avoiding the inconvenience of re-sterilisation.

In practice, a plurality of different versions of gripper tool 30 would be provided, having different length arms 34. The arms 34 may range from approximately 5 to approximately 10 centimetres, the lens of each tool 30 being selected so as to have a point of focus at the ends 36 of the gripper arms 34. Thus, depending upon the application, a tool with suitable length arms can be chosen, without any need to adjust lenses or the like.

It is envisaged that the tool 30 would be provided in a sealed package already sterilised, for example by irradiation, such that a package containing the desired tool can be selected, the package opened and tool immediately used. Following usage, the tool is discarded.

Also shown in Figure 3 is an embodiment of mould, which includes a main mould body 40 surrounding the body portion 32 of the tool 30, first and second movable tool arm mould portions 42,44, each having a recess shaped to form the outer surface of a tool arm, and a core pin 46 which extends substantially coaxially into the cavity of the main mould body 40 and mould portions 42,44 and which in use forms the interior surfaces of the tool body 32 and tool arms 34. In the annular space between the core pin 46 and the main mould body 40, an ejector sleeve 48 is reciprocally slidable and includes suitable nozzles (not shown) for injecting plastics material into the mould and for forming the recessed annular shoulder 36 of the tool body 32.

A fastening screw 50 is also provided for fastening together the mould portions 42,44.

## Claims

1. A magnifying tool (10) comprising a tubular support member (12) which includes first and second elongate cavities (18) at opposing sides of a first end of the support member, first and second tool arms (20) insertable into and securable in the first and second cavities respectively, and a magnifying lens (22) supported by the tubular support member and having its point of focus oriented along the axis of the tubular support member.

2. A magnifying tool according to claim 1, wherein the first and second cavities (18) and/or the first and second arms (20) include releasable fastening means for allowing the first and second arms to be removed from the tubular support.

3. A magnifying tool according to claim 2, wherein the fastening means includes a burr and a slot on respective ones of the tool arms and elongate cavities.

4. A magnifying tool according to claim 1, wherein the first and second tool arms (20) are permanently fixable in their respective cavities (18).

5. A magnifying tool according to any preceding claim, wherein the lens (22) is securable to a second end of the tubular support (12) opposite to the first end.

6. A magnifying tool according to any preceding claim, wherein the tubular support (12) is formed from inner and outer tubular members (14,16) the inner member (16) being insertable into the outer member (14); one of the inner and outer members including two elongate channels (18) therein which, when the inner and outer members are assembled, form the first and second elongate cavities with a surface of the other of the inner and outer tubular members.

7. A magnifying tool according to claim 6, wherein the channels (18) are formed in the inner tubular member (16).

8. A magnifying tool according to claim 6 or 7, wherein the inner tubular member (16) is shorter than the outer tubular member (14) and, when inserted in the outer tubular member provides a shoulder delimited by a portion of an inner wall of the second end of the outer tubular member (14).

9. A magnifying tool according to any preceding claim, wherein the tool arms (20) are tweezer arms, pincer arms or arms of any other gripping tool.

10. A magnifying tool according to any preceding claim, wherein the ends of the arms are pointed so as to enable them to pierce a surface worked upon by the tool.

11. A medical gripping tool comprising, formed from a single unit, a tubular body member (30) and a pair of gripper arms (34) extending from opposing sides of a first end of the body member, each gripper arm (34) having a pointed end (36); a lens being securable to a second end of the body member opposite to the first end, so as to have its axis of focus extending along the gripper arms.

12. A medical gripping tool according to claim 11, wherein the body member (30) and gripper arms (34) are moulded from a single moulding.

13. A medical gripping tool according to claim 11 or 12, wherein the gripper arms (34) are in the form of pivotless extensions of the body member.

14. A medical gripping tool according to claim 11, 12 or 13, wherein the ends of the gripper arms (34) are in the form of tweezer and have substantially flat opposing faces for assisting in gripping.

15. A medical gripping tool according to any one of claims 11 to 14, wherein the body member (30) includes a recessed annular portion (36) at its second end for receiving a lens.

16. A medical gripping tool according to any one of claims 11 to 15, wherein the gripping tool is made of a plastics material.
